(19) Europäisches Patentamt / European Patent Office / Office européen des brevets

(11) EP 3 178 942 A1

(12) EUROPEAN PATENT APPLICATION
published in accordance with Art. 153(4) EPC

(43) Date of publication:
14.06.2017 Bulletin 2017/24

(51) Int Cl.:
C12Q 1/68 (2006.01)  C12Q 1/04 (2006.01)
G01N 21/27 (2006.01)  G01N 33/48 (2006.01)
G01N 33/49 (2006.01)

(21) Application number: 15829324.1

(22) Date of filing: 19.06.2015

(86) International application number:
PCT/JP2015/067711

(87) International publication number:
WO 2016/021310 (11.02.2016 Gazette 2016/06)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 05.08.2014 JP 2014159345

(71) Applicant: Fujifilm Corporation
Minato-ku
Tokyo 106-8620 (JP)

(72) Inventors:
• FUKUI, Shinichiro
Kanagawa 258-8538 (JP)
• ISHII, Yasuyuki
Kanagawa 258-8538 (JP)
• MATSUBARA, Kenta
Kanagawa 258-8538 (JP)

(74) Representative: Klunker . Schmitt-Nilson . Hirsch
Patentanwälte
Destouchesstraße 68
80796 München (DE)

(54) METHOD FOR TESTING FETAL CHROMOSOME

(57) Provided is a method for inspecting a chromosome of a fetus in which fetus-derived nucleated red blood cells are reliably obtained from maternal blood and are efficiently inspected within a short period of time. The method includes: a collection step of obtaining maternal blood from a pregnant mother; a concentration step of concentrating nucleated red blood cells in the maternal blood; a sorting step of sorting the nucleated red blood cells in the maternal blood, in which the nucleated red blood cells are concentrated through the concentration step, into mother-derived nucleated red blood cells and fetus-derived nucleated red blood cells in accordance with the shapes of nuclei and spectral characteristics at wavelengths included in a light wavelength region of 400 to 650 nm; an amplification step of amplifying a nucleic acid of a chromosome of the nucleated red blood cells; a definition step of defining an amount of the amplified product of the nucleated red blood cells; and a determination step of determining presence or absence of a numerical abnormality of the fetus-derived chromosome.

FIG. 1

COLLECTION STEP — S12

CONCENTRATION STEP — S14

SORTING STEP — S16

AMPLIFICATION STEP — S18

DEFINITION STEP — S20

DETERMINATION STEP — S22

**Description**

**BACKGROUND OF THE INVENTION**

1. Field of the Invention

The present invention relates to a method for inspecting a chromosome of a fetus, and particularly to a method for inspecting a chromosome of a fetus in which the inspection is performed using fetus-derived red blood cells collected from blood of a pregnant mother.

2. Description of the Related Art

In the related art, an amniotic fluid test of checking a chromosome of a fetal cell in amniotic fluid has been performed through amniocentesis as a prenatal diagnosis. However, in this method, a possibility of miscarriage is pointed out as a significant problem.

In contrast, it has been recently found that a fetal cell is transferred into blood of a pregnant mother (hereinafter, also simply referred to as a "mother") and circulates with blood in the mother. Therefore, it is possible to realize a safe prenatal diagnosis having a low possibility of miscarriage if it is possible to reliably analyze deoxyribonucleic acid (DNA) of a chromosome of a fetal cell in blood of the mother with excellent reproducibility, using maternal blood.

However, approximately only one fetal cell (nucleated red blood cell) existing in maternal blood exists in the number (mL) of maternal blood (1 mL = $10^{-6}$ $m^3$). Reliably obtaining such an extremely a small number of fetal cells (nucleated red blood cells) is a significantly large problem in a case of performing a prenatal diagnosis using maternal blood.

Regarding such a problem, in JP2010-525832A and Wakako SUZUKI, et al., "Enrichment and Automated Detection of NRBC from Maternal Blood", The Showa University Society, vol. 72, No. 4 (pp. 471 to 478, 2012) described below, nucleated red blood cells as fetal cells in maternal blood are obtained through a method for concentrating nucleated red blood cells, for example, a technique of removing plasma components and red blood cell components of a mother through density gradient centrifugation, a technique (magnetic activated cell sorting: MACS method) of magnetically separating white blood cells of a mother from blood using an antibody which immunologically reacts specifically to proteins on surfaces of the white blood cells, a technique (fluorescence activated cell sorting: FACS method) of separating nucleated red blood cells of a fetus from blood using a fluorescent coloring agent and an antibody which immunologically reacts specifically to a γ chain of fetal hemoglobin, and the like. These methods are useful techniques for increasing the probability of obtaining target cells by concentrating fetus-derived nucleated red blood cells.

In addition, a technique of obtaining required cells after identifying the types of cells using optical information is disclosed. For example, it is disclosed in JP2002-514304A that an absorption image of transmitted visible light is generated after dyeing cytoplasm, a fluorescent image of a nucleus is formed after emitting excitation light, and nucleated red blood cells are discriminated using contrast images of cytoplasm and nuclei. In addition, a blood cell type-discriminating apparatus which discriminates the types of blood cells after performing measurement using light at a wavelength in the vicinity of a maximum wavelength region of hemoglobin is disclosed in JP1983-115346A (JP-S58-115346A). A biological information analysis apparatus in which optical characteristics of biological tissue are used is disclosed in JP2014-14485A. A method for concentrating fetus-derived nucleated red blood cells and identifying fetus-derived nucleated red blood cells from information of the forms of cells is disclosed in D. Cha et al., "A simple and sensitive erythroblast scoring system to identify fetal cells in maternal blood", Prenat Diagn 2003; 23: 68 to 73.

A method for amplifying a chromosome of a fetal cell and defining the amount of the amplified product to detect a disease caused by chromosome imbalance is disclosed in JP2004-531271A, as a method for detecting a chromosome abnormality in a fetus after obtaining fetal cells (nucleated red blood cells).

**SUMMARY OF THE INVENTION**

Various methods for selecting fetus-derived nucleated red blood cells from maternal blood of a pregnant mother have been reviewed in this manner. However, a method for reliably obtaining fetus-derived nucleated red blood cells after separating the fetus-derived nucleated red blood cells from mother-derived nucleated red blood cells is required in order to efficiently perform a prenatal diagnosis. Although the methods disclosed in the above-described related art documents can increase the probability of obtaining fetus-derived nucleated red blood cells from maternal blood, it is impossible to find whether the obtained nucleated red blood cells are mother-derived nucleated red blood cells or fetus-derived nucleated red blood cells, until chromosomes are inspected. Accordingly, in a case where cells which have been inspected are mother-derived nucleated red blood cells, it is necessary to inspect chromosomes until fetus-derived nucleated red blood cells are sorted out, and therefore, it takes time to perform the inspection again.

The present invention has been made in consideration of such circumstances, and an object of the present invention is to provide a method for inspecting a chromosome of a fetus in which it is possible to reliably obtain fetus-derived nucleated red blood cells from maternal blood and to efficiently inspect the fetus-derived nucleated red blood cells within a short period of time.

In order to achieve the purpose of the present invention, according to an aspect of the present invention, there is provided a method for inspecting a chromosome of a fetus, the method comprising: a collection step of obtaining maternal blood from a pregnant mother; a concentration step of concentrating nucleated red blood cells in the maternal blood; a sorting step of sorting the nucleated red blood cells in the maternal blood, in which the nucleated red blood cells are concentrated through the concentration step, into mother-derived nucleated red blood cells and fetus-derived nucleated red blood cells in accordance with shapes of nuclei and spectral characteristics at wavelengths included in a light wavelength region of 400 to 650 nm; an amplification step of amplifying a nucleic acid of a chromosome of at least the fetus-derived nucleated red blood cells; a definition step of defining an amount of the amplified product of at least the fetus-derived nucleated red blood cells which has been amplified through the amplification step; and a determination step of determining presence or absence of a numerical abnormality of the fetus-derived chromosome through comparison of the defined amount of the amplified product with an amount of an amplified product of a chromosome of a mother or an amount of an amplified product of a chromosome of a fetus which is known that there is no abnormality.

According to the method for inspecting a chromosome of a fetus of the present invention, it is possible to reliably sorting out the fetus-derived nucleated red blood cells, by sorting the mother-derived nucleated red blood cells and the fetus-derived nucleated red blood cells contained in the pregnant mother in accordance with the shape of a nucleus and spectral characteristics in a light wavelength region of 400 to 650 nm. Accordingly, it is possible to efficiently reliably perform the inspection of a chromosome of a fetus using a gene using maternal blood.

In another aspect of the present invention, it is preferable that the sorting step includes a first sorting step of sorting out a candidate of a nucleated red blood cell in accordance with at least the shapes of the nuclei, and a second sorting step of sorting out the candidate of the nucleated red blood cell which has been sorted out through the first sorting step, in accordance with the spectral characteristics.

According to this aspect, it is possible to reliably sort out the fetus-derived nucleated red blood cells by sorting the nucleated red blood cells through different methods since the sorting step includes the first sorting step including a step of sorting out a candidate of a nucleated red blood cell in accordance with the shape of a nucleus and the second sorting step of sorting a candidate of a nucleated red blood cell in accordance with spectral characteristics. In addition, in the first sorting step, it is possible to remove red blood cells having no nucleus by performing the sorting in accordance with the shape of a nucleus and to sort the nucleated red blood cells. After sorting the nucleated red blood cells, it is possible to efficiently sort out the fetus-derived nucleated red blood cells by sorting out the fetus-derived nucleated red blood cells and the mother-derived nucleated red blood cells in accordance with spectral characteristics.

In still another aspect of the present invention, it is preferable that the first sorting step includes a step of selecting a candidate of a nucleated red blood cell satisfying Formula (1) (also referred to as Formula 1) and Formula (2) (also referred to as Formula 2), in a case where an area of cytoplasm of a cell which becomes the candidate of the nucleated red blood cell is set to C, an area of a nucleus of a cell which becomes the candidate of the nucleated red blood cell is set to N, and a length of a diameter of a circular shape circumscribing the nucleus of the cell which becomes the candidate of the nucleated red blood cell or a length of a major axis of an elliptical shape thereof is set to L.

$$0.25 < N / C < 1.0 \qquad (1)$$

$$0.65 < N / (L \times L) < 0.785 \qquad (2)$$

In this aspect, specific conditions for the sorting in accordance with the shape of a nucleus are prescribed. Formula (1) prescribes an area ratio of cytoplasm and a nucleus and Formula (2) prescribes a degree of circularity of a nucleus. Since there is a high possibility that a cell having a nucleus under the conditions of the above-described Formula (1) and the above-described Formula (2) may be a red blood cell, it is possible to increase a possibility of selecting a fetus-derived nucleated red blood cell through the sorting performed in accordance with the spectral characteristics.

In still another aspect of the present invention, it is preferable that the first sorting step further includes two steps including a first step of extracting cells which possibly has a nucleus, and a second step of sorting out the candidate of the nucleated red blood cell from the cells extracted in the first step using Formula (1) and Formula (2).

According to this aspect, the first sorting step further includes two steps including the first step of performing rough sieving by extracting the cell which possibly has a nucleus, and a second step of performing sorting using shape information based on the above-described Formula (1) and the above-described Formula (2), and therefore, it is possible to more reliably sort out the fetus-derived nucleated red blood cell.

As an example of the method for "extracting a cell which possibly has a nucleus" in the first step, brightness information of an image which has been obtained by imaging a cell with white light is binarized with a predetermined threshold value, and dot-shaped and island-shaped continuous regions are extracted from the binarized image. Among the continuous regions, a region of which the size is 1 to 5 $\mu$m is set as a region which is possibly a cell nucleus, and a cell including the region is set as a cell which possibly has a cell nucleus. For example, the major axis of a minimum ellipse circumscribing the continuous regions can be set as the size. The size of the binarized image may be obtained after arranging the shape of the continuous regions by performing morphology processing such as erosion and dilation.

In still another aspect of the present invention, it is preferable that the spectral characteristics include an absorption coefficient.

By using the absorption coefficients as the spectral characteristics, it is possible to perform sorting using the difference in the absorption coefficients between oxygenated hemoglobin and reduced hemoglobin contained in a red blood cell.

In still another aspect of the present invention, it is preferable that, in the sorting step, an absorbance of the nucleated red blood cell is measured at each of at least two or more wavelengths including a wavelength in a first light wavelength region, in which an absorbance of the fetus-derived nucleated red blood cell exceeds an absorbance of the mother-derived nucleated red blood cell, and a wavelength in a second light wavelength region in which the absorbance of the mother-derived nucleated red blood cell exceeds the absorbance of the fetus-derived nucleated red blood cell.

According to this aspect, first, it is possible to first perform sorting whether a nucleated red blood cell (a cell which becomes a candidate of a fetus-derived nucleated red blood cell) to be sorted out has an absorption coefficient of hemoglobin, and therefore, it is possible to exclude a white blood cell-derived cell which does not have an absorption coefficient of hemoglobin. Next, in a cell having an absorption coefficient of hemoglobin, the magnitude of the absorbance of the fetus-derived nucleated red blood cell and the absorbance of the mother-derived nucleated red blood cell are reversed in the absorbance at each wavelength, by measuring wavelengths used in the sorting step at each wavelength selected from a first wavelength region in which the absorbance of the fetus-derived nucleated red blood cell is higher than that of the mother-derived nucleated red blood cell, and a second wavelength region in which the absorbance of the mother-derived nucleated red blood cell is higher than that of the fetus-derived nucleated red blood cell. Accordingly, it is possible to clarify the difference between the mother-derived nucleated red blood cell and the fetus-derived nucleated red blood cell by comparing the absorbances measured at each wavelength with each other, and therefore, it is possible to sort out the mother-derived nucleated red blood cell and the fetus-derived nucleated red blood cell. Through the comparison of the absorbances with each other, the difference in the proportion of the absorbance is increased after obtaining the proportion of the absorbance measured at each wavelength, and therefore, it is possible to perform the sorting.

It is possible to reduce an influence of noise by measuring the absorbance at each of three or more wavelengths. In a case of using three or more wavelengths, it is possible to reduce an influence of noise by measuring the absorbance at a plurality of wavelengths obtained by shifting wavelengths and setting an average value thereof as an absorbance to be used for the sorting.

In still another aspect of the present invention, it is preferable that, in the sorting step, a possibility of being a fetus-derived nucleated red blood cell is prioritized based on difference in the absorbances of the mother-derived nucleated red blood cell and the fetus-derived nucleated red blood cell which have been measured at each of two or more wavelengths.

According to this aspect, it is possible to obtain the difference in the absorbance between the fetus-derived nucleated red blood cell and the mother-derived nucleated red blood cell at each wavelength by measuring the absorbance using two or more wavelengths. Accordingly, it is possible to prioritize the possibility of being a fetus-derived nucleated red blood cell from the difference in these absorbances. Therefore, it is possible to accurately sort out the fetus-derived nucleated red blood cell from candidate cells which are a plurality of nucleated red blood cells, by sorting out the fetus-derived nucleated red blood cell based on this priority.

In still another aspect of the present invention, it is preferable that the first light wavelength region is a wavelength region exceeding 450 nm and less than 480 nm, and the second light wavelength region is a wavelength region exceeding 550 nm and less than 575 nm.

In this aspect, the first light wavelength region and the second light wavelength region which are used for measuring the spectral characteristics are specifically prescribed, and therefore, it is possible to reverse the magnitude of the absorbances using the wavelengths of the light wavelength regions in this range and to clarify the difference in optical characteristics between the mother-derived nucleated red blood cell and the fetus-derived nucleated red blood cell.

In still another aspect of the present invention, it is preferable that the concentration step is performed through density gradient centrifugation.

According to this aspect, it is possible to increase the concentration of nucleated red blood cells in maternal blood without destroying cells, by performing the concentration step through density gradient centrifugation.

According to the method for inspecting a chromosome of a fetus of the present invention, it is possible to reliably sort out a mother-derived nucleated red blood cell and a fetus-derived nucleated red blood cell, and therefore, it is possible to reliably efficiently perform gene inspection of a fetus using maternal blood.

**BRIEF DESCRIPTION OF THE DRAWINGS**

Fig. 1 is a flow chart showing a procedure of a method for inspecting a chromosome of a fetus.
Fig. 2 is a flow chart showing a procedure of a first sorting step in a sorting step.
Fig. 3A is a schematic view showing the shape of a cell to be selected.
Fig. 3B is a schematic view showing the shape of a cell to be removed.
Fig. 4 is a graph showing absorption coefficients with respect to wavelengths of reduced hemoglobin (Hb) and oxygenated hemoglobin (HbO$_2$).
Fig. 5 is a flow chart showing a procedure of a first embodiment of sorting performed in accordance with optical characteristics.
Fig. 6 is an explanatory view illustrating a method for selecting a wavelength during measurement of an absorbance.
Fig. 7 is a view illustrating a method for setting a search range based on the number of reference red blood cells.
Fig. 8 is a flow chart showing a procedure of setting the search range based on the number of reference red blood cells.
Fig. 9 is a view illustrating a method for setting a search range of reference red blood cells based on the area of a nucleated red blood cell.
Fig. 10 is a flow chart showing a procedure of setting the search range of reference red blood cells based on the area of the nucleated red blood cell.
Fig. 11 is a flow chart showing a procedure of a second embodiment of sorting performed in accordance with optical characteristics.

**DESCRIPTION OF THE PREFERRED EMBODIMENTS**

Hereinafter, a method for inspecting a chromosome of a fetus according to the present invention will be described with reference to accompanying drawings. "To" in the present specification is used as a meaning including numerical values described before and after "to" as lower limit values and upper limit values.

[Method for Inspecting Chromosome of Fetus]

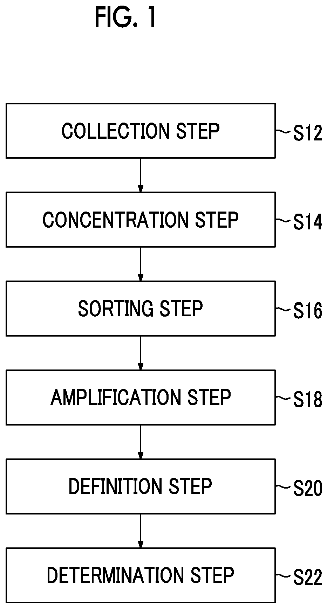

Fig. 1 is a flow chart showing a procedure of a method for inspecting a chromosome of a fetus in the present embodiment. The method for inspecting a chromosome of a fetus of the present embodiment includes a collection step (Step S12) of collecting maternal blood from a pregnant mother; a concentration step (Step S14) of concentrating nucleated red blood cells in the maternal blood; a sorting step (Step S16) of sorting the nucleated red blood cells in the maternal blood, in which the nucleated red blood cells are concentrated through the concentration step, into mother-derived nucleated red blood cells and fetus-derived nucleated red blood cells in accordance with the shape of a nucleus and spectral characteristics in a light wavelength region of 400 to 650 nm; an amplification step (Step S18) of amplifying a nucleic acid of the chromosome of at least the fetus-derived nucleated red blood cells; a definition step (Step S20) of defining the amount of the amplified product of at least the fetus-derived nucleated red blood cells which has been amplified through the amplification step; and a determination step (Step S22) of determining the presence or absence of a numerical abnormality of the fetus-derived chromosome through comparison of the defined amount of the amplified product with the amount of an amplified product of a chromosome of a mother or the amount of an amplified product of a chromosome of a fetus which is known that there is no abnormality. Hereinafter, each step will be described.

<collection Step (Step S12)>

The collection step is a step of collecting maternal blood which is a blood sample. It is preferable to use peripheral blood of a pregnant mother having no concern of invasion, as maternal blood.
Mother-derived nucleated red blood cells and fetus-derived nucleated red blood cells are contained in peripheral blood of a mother in addition to white blood cells, such as eosinophils, neutrophils, basophils, monocytes, and lymphocytes, which are derived from a mother, or mature red blood cells having no nucleus. It is known that the fetus-derived nucleated red blood cells exist in maternal blood after about 6 weeks of pregnancy. In the present embodiment in which a prenatal diagnosis is performed, peripheral blood of a mother after about 6 weeks of pregnancy is inspected.
Fetus-derived nucleated red blood cells are erythrocyte precursors which exist in blood of a mother after passing through the placenta. Red blood cells of a fetus can be nucleated during pregnancy of a mother. Since there are chromosomes in these red blood cells, it is possible to obtain fetus-derived chromosomes and fetus genes through less invasive means. It is known that these fetus-derived nucleated red blood cells exist at a ratio of one fetus-derived nucleated red blood cell to $10^6$ cells in maternal blood. Therefore, the existence probability of the fetus-derived nucleated red blood cells in peripheral blood of a mother is significantly low.

<Concentration Step (Step S14)>

Next, concentration of nucleated red blood cells in maternal blood collected in the collection step is performed through a concentration step. The concentration step is preferably performed through density gradient centrifugation.

[Density Gradient Centrifugation]

The density gradient centrifugation is a method for performing separation using the difference in the density of components in blood. By selecting a first medium and a second medium so as to interpose density values of components to be separated, it is possible to collect target components (fetus-derived nucleated red blood cells in the present embodiment) at a boundary between the first medium and the second medium after the centrifugation. Moreover, it is possible to obtain blood in which red blood cells are concentrated, by collecting fractions existing at a boundary between the first medium and the second medium.

Specifically, the concentration of nucleated red blood cells can be performed through a method including a step of injecting the first medium into a bottom portion of a centrifuge tube, a step of cooling the centrifuge tube in which the first medium is stored, a step of stacking the second medium on the first medium which has been cooled within the centrifuge tube, a step of stacking peripheral blood of a mother, as a blood sample, on the second medium within the centrifuge tube, a step of subjecting the first medium, the second medium, and the blood sample which have been stored in the centrifuge tube, to centrifugation, and a step of collecting fractions containing fetus-derived nucleated red blood cells from a layer between the first medium and the second medium after the centrifugation.

The density of blood of a mother which contains nucleated red blood cells of a fetus is disclosed in WO2012/023298A. According to the disclosure, the density of fetus-derived nucleated red blood cells which is assumed is about 1.065 to 1.095 g/mL, the density of blood cells of a mother is about 1.070 to 1.120 g/mL in a case of red blood cells and about 1.090 to 1.110 g/mL in a case of eosinophils, about 1.075 to 1.100 g/mL in a case of neutrophils, about 1.070 to 1.080 g/mL in a case of basophils, about 1.060 to 1.080 g/mL in a case of lymphocytes, and about 1.060 to 1.070 g/mL in a case of monocytes. It is easy to convert the numerical values of the density from a unit represented by grams per milliliter (g/mL) to an SI unit system, and 1 gram per milliliter (g/mL) is 1000 kilograms per cubic meter ($kg/m^3$).

The density of media (first medium and second medium) is set so as to separate fetus-derived nucleated red blood cells having a density of 1.065 to 1.095 g/mL from other blood cell components in a mother. The density of the center of the fetus-derived nucleated red blood cells is about 1.080 g/mL. Therefore, it is possible to collect desired fetus-derived nucleated red blood cells at a boundary of the density by producing two media which have different densities interposing the density and making the media adjacently overlap each other. It is preferable to set the density of the first medium to 1.08 g/mL to 1.10 g/mL and the density of the second medium to 1.06 g/mL to 1.08 g/mL. More preferably, the density of the first medium is 1.08 g/mL to 1.09 g/mL and the density of the second medium is 1.065 g/mL to 1.08 g/mL. As a specific example, it is possible to separate plasma components, eosinophils, and monocytes from desired fractions to be collected, by setting the density of the first medium to 1.085 g/mL and the density of the second medium to 1.075 g/mL. In addition, it is also possible to partially separate red blood cells, neutrophils, and lymphocytes therefrom. In the present invention, the types of the first medium and the second medium may be the same as or different from as each other, and are not limited as long as it is possible to realize the effect of the invention. However, use of the same types of media is a preferred aspect.

As the first medium and the second medium used in the concentration step, it is preferable to use media such as Percoll (registered trademark) which is a silica colloid particle dispersion liquid which has a diameter of 15 to 30 nm and is coated with polyvinyl pyrrolidone, Ficoll (registered trademark)-Paque which is a neutral hydrophilic polymer rich in a side chain made of sucrose, and Histopaque (registered trademark) which is a solution made of polysucrose and sodium diatrizoate. In the present embodiment, it is possible to preferably use Percoll (registered trademark) and Histopaque (registered trademark). A product of Percoll (registered trademark) having a density (specific gravity) of 1.130 is commercially available and it is possible to prepare a medium having a target density (specific gravity) by diluting the product. It is possible to prepare a first medium and a second medium to have a desired density using a medium having a density of 1.077 and a medium having a density of 1.119 as commercially available Histopaque (registered trademark).

[Other Concentration Methods]

As a method for concentrating nucleated red blood cells, it is possible to concentrate nucleated red blood cells by separating white blood cells from the nucleated red blood cells using an antibody specifically bound to CD45 (CD: cluster of differentiation) (a classification number of a monoclonal antibody based on CD classification) which is an antigen of a usual white blood cell, using a magnetic effect, in addition to the above-described density gradient centrifugation.

<Sorting Step (Step S16)>

The sorting step is a step of sorting nucleated red blood cells in maternal blood, in which the nucleated red blood cells are concentrated through the concentration step into mother-derived nucleated red blood cells and fetus-derived nucleated red blood cells in accordance with the shape of a nucleus and spectral characteristics.

Other blood components, such as white blood cells, other than the nucleated red blood cells are also contained in peripheral blood containing the nucleated red blood cells concentrated in the concentration step. The blood components such as white blood cells can be removed from maternal blood through the concentration step. However, it is difficult to completely remove the blood cells. Therefore, in the sorting step, mother-derived nucleated red blood cells and fetus-derived nucleated red blood cells are sorted out through a first sorting step in which the sorting is performed in accordance with the shape of a nucleus and a second sorting step in which the sorting is performed in accordance with spectral characteristics.

The sorting step is performed using a specimen for analysis which has been produced by coating a transparent substrate, preferably slide glass, with peripheral blood containing the nucleated red blood cells concentrated in the concentration step.

(Sorting Using Information of Shape of Cell)

A nucleated red blood cell which becomes a candidate of a fetus-derived nucleated red blood cell is sorted out in accordance with information of the shape of a cell (hereinafter, also referred to as the "shape of a cell") using the above-described specimen for analysis.

Examples of the sorting performed in accordance with the information of the shape of a cell include the ratio of the area of a nuclear region to the area of cytoplasm, the degree of circularity of a nucleus, the area of a nuclear region, the brightness of a nucleus, and a crushed condition of a nucleus. Among these, it is preferable to sort a cell, which has conditions satisfying the ratio of the area of a nuclear region to the area of cytoplasm and the degree of circularity of a nucleus, as a candidate of fetus-derived nucleated red blood cell. The area of cytoplasm and the area of a nuclear region are areas on a face on which the specimen for analysis is observed.

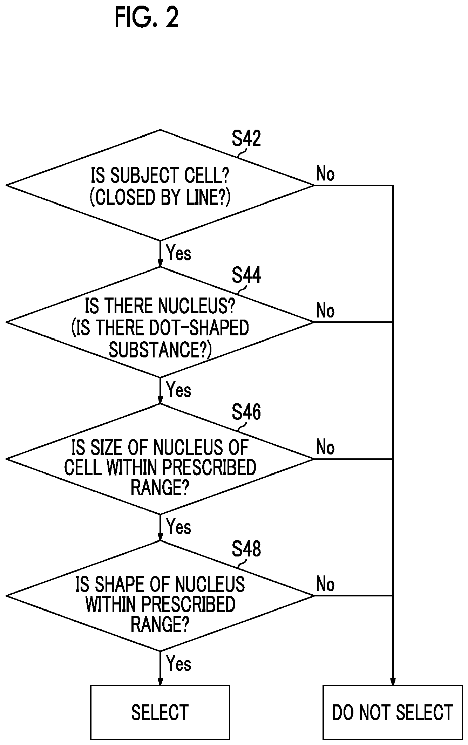

Fig. 2 is a flow chart showing a procedure of an example of a first sorting step of performing sorting in accordance with the shape (shape of a nucleus) of a cell in a sorting step.

In the sorting performed in accordance with the shape of a cell, cells are first extracted (Step S32). Determination of whether or not a subject is a cell is performed using a binary image. A binary image of brightness information of an image which has been obtained by imaging a specimen for analysis with a white light source is generated using a predetermined threshold value, and a subject having dot-shaped and island-shaped continuous regions, that is, regions closed by an arbitrary closed curve are extracted as cells from the generated binarized image.

Next, the cells extracted in Step S32 are selected depending on the presence or absence of a nucleus (Step S34). The presence or absence of a nucleus is sorted out depending on whether or not there is a nucleus (dot-shaped substance) in a cell. It is also possible to use the binary image in a case of determining the presence or absence of a nucleus. In a case where there is a region, which is closed by a closed curve and has an area smaller than that of the region extracted as a cell, in the region extracted as a cell, it is possible to extract the region as a nucleus.

Next, cells having 1 to 5 $\mu$m of a size (the size of dot-shaped and island-shaped continuous regions) of a nucleus are extracted (Step S36). The selection of cells having a nucleus with a size of 1 to 5 $\mu$m is effective for sorting nucleated red blood cells. The closed curve indicating the outer shape of a nucleus can be approximated to a circular shape or an elliptical shape and the "size of the dot-shaped and island-shaped continuous regions" can be set as a diameter of the circular shape, to which the outer shape of a nucleus is approximated, or a major axis of the elliptical shape, to which the outer shape of a nucleus is approximated.

Examples of the circular shape to which the outer shape of a nucleus is approximated include a circular shape having a minimum diameter, out of circular shapes circumscribing the closed curve indicating the outer shape of a nucleus. Examples of the elliptical shape to which the outer shape of a nucleus is approximated include an elliptical shape having a minimum major axis, out of elliptical shapes circumscribing the closed curve indicating the outer shape of a nucleus. Cells having 1 to 5 $\mu$m of a diameter of the circular shape to which the outer shape of a nucleus is approximated or a major axis of the elliptical shape to which the outer shape of a nucleus is approximated are extracted.

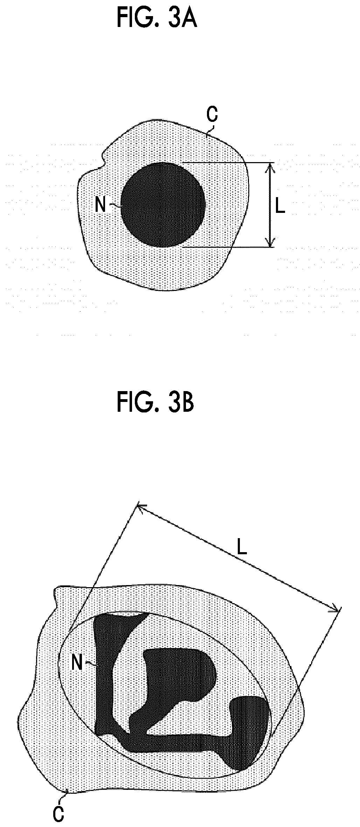

Next, cells are sorted in accordance with the shape of a nucleus (Step S38). Fig. 3A is a schematic view showing the shape of a cell to be selected, and Fig. 3B is a schematic view showing the shape of a cell to be removed. A method for performing sorting in accordance with the ratio of the area of a nuclear region to the area of cytoplasm and a method for performing sorting in accordance with the degree of circularity of a nucleus are described as specific examples of sorting performed in accordance with the shape of a nucleus. A cell in a case where the ratio of the area of a nuclear region to the area of cytoplasm satisfies the following Formula (1) is selected as a nucleated red blood cell which becomes a candidate of a fetus-derived nucleated red blood cell. In a case where the area of cytoplasm of a cell is set to C and the area of a nucleus of the cell is set to N, a cell having a nucleus of which the area of N to C exceeds 0.25 and less than 1.0 is selected as a nucleated red blood cell which becomes a candidate of a fetus-derived nucleated red blood cell.

$$0.25 < (N / C) < 1.0 \qquad (1)$$

In addition, examples of the case satisfying the selection conditions of the degree of circularity of a nucleus include a case of satisfying the following Formula (2). In a case where the diameter of a nucleus or the major axis of a nucleus is set to L, a cell having a nucleus of which the ratio of the area of the nucleus to the square of the diameter of the nucleus or the major axis of the nucleus exceeds 0.65 and less than 0.785 is selected as a nucleated red blood cell which becomes a candidate of a fetus-derived nucleated red blood cell. The diameter of the nucleus or the major axis of the nucleus can be obtained through the same method as that in the case where the size of a nucleus is obtained in Step S34.

$$0.65 < (N / (L \times L)) < 0.785 \qquad (2)$$

In the sorting performed in accordance with the shape of a cell, it is possible to reduce the number of nucleated red blood cells which become candidates of fetus-derived nucleated red blood cells on which sorting performed through image processing is to be performed, by performing rough sieving in the first sorting step in accordance with Step S32 to Step S36 and performing sorting through the shape of a nucleus in the second step in accordance with Step S38, and therefore, it is possible to efficiently sort out nucleated red blood cells which become candidates of fetus-derived nucleated red blood cells.

In the sorting performed in accordance with the shape, it is necessary to satisfy all of the conditions of Step S32 to Step S38. Cells which do not satisfy any one of the conditions of Step S32 to Step S38 are not selected as nucleated red blood cells which become candidates of fetus-derived nucleated red blood cells.

Configuration examples of a system of sorting out nucleated red blood cells which become candidates of fetus-derived nucleated red blood cells using the information of the shape of a cell include a system equipped with an optical microscope, a digital camera, a stage for slide glass, an optical transfer system, an image processing personal computer (PC), a control PC, and a display. The optical transfer system includes an objective lens and a charge coupled device (CCD) camera. Examples of the image processing PC include a configuration including a processing system of performing analysis and storing data. Examples of the control PC include a configuration of including a control system of controlling the position of storage for slide glass or controlling the entire processing.

(Sorting Performed in accordance with Optical Characteristics)

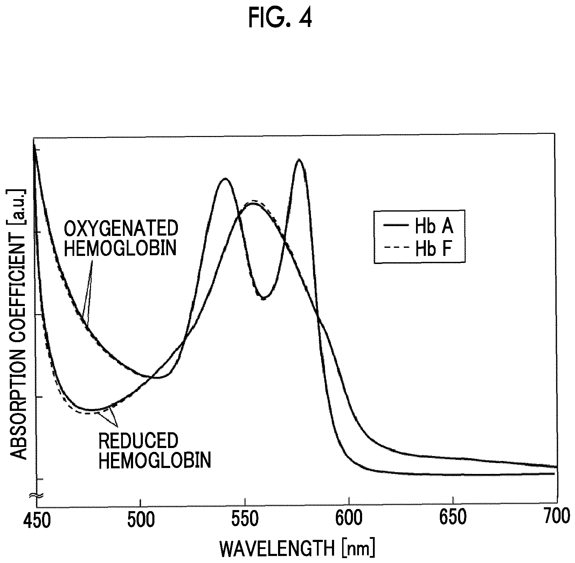

In a case of performing the sorting in accordance with optical characteristics, it is possible to use spectral characteristics caused by difference in oxygen affinity of hemoglobin contained in red blood cells. Fig. 4 is a graph (which is disclosed in JP2014-1485A) showing absorption coefficients with respect to wavelengths of reduced hemoglobin (Hb) and oxygenated hemoglobin ($HbO_2$). Here, the absorption coefficients are constants indicating how much light is absorbed by a certain medium on which light is incident. It is known that the absorption coefficients change depending on interaction with oxygen as shown in Fig. 4. That is, oxygenated hemoglobin has a red tone and blueness is exhibited in accordance with oxygenated hemoglobin becoming reduced hemoglobin.

In the sorting performed in accordance with optical characteristics, mother-derived nucleated red blood cells and fetus-derived nucleated red blood cells are sorted out using the difference in oxygen affinity of hemoglobin of the fetus-derived nucleated red blood cells from red blood cells of a mother and slightly existing mother-derived nucleated red blood cells. For example, mother-derived nucleated red blood cells and fetus-derived nucleated red blood cells are sorted out using the difference in spectral characteristics between nucleated red blood cells and red blood cells existing in the vicinity of these nucleated red blood cells using blood components with which a glass substrate is coated.

In the spectral characteristics, the difference in a wavelength region, in which there is a difference in absorption coefficients between reduced hemoglobin and oxygenated hemoglobin, is detected. Hemoglobin in red blood cells of an adult is a tetramer $\alpha2\beta2$ (HbA). In contrast, hemoglobin in red blood cells of a fetus is tetramer $\alpha2\gamma2$ (HbF) formed of two $\alpha$ chains and two $\gamma$ chains, and it is known that $\alpha2\gamma2$ is replaced with HbA2 formed of HbA occupying the majority of hemoglobin and small amount of HbF, after birth. In placenta, hemoglobin of a fetus obtains oxygen which is received from hemoglobin (HbA) in blood of a pregnant mother. Since hemoglobin (HbA) contained in red blood cells in blood of an adult whereas fetus-type hemoglobin (HbF) is tetramer $\alpha2\gamma2$, fetus-type hemoglobin has characteristics in that oxygen affinity of HbF is higher than that of HbA. In a prenatal diagnosis, it is preferable to perform inspection after collecting peripheral blood of a pregnant mother as a blood sample. Peripheral blood is generally collected from the vein and the amount of oxygen in blood in the vein is more deficient than that of oxygen in the artery. A normal value of the degree of oxygen saturation in central venous blood in a healthy person is known as 60% to 80% and the degree of oxygen saturation is increased and decreased depending on demand of oxygen of the whole body. The oxygen affinity differs in HbA and HbF, and therefore, the oxygen binding amount differs in HbA and HbF even in venous blood. Thus, there is a characteristic that the oxygen binding amount of HbF becomes higher than that of HbA.

There is a difference in oxygen affinity in the vein even in hemoglobin (HbA) of a mother and hemoglobin (HbF) of a fetus, and therefore, there is a difference in absorption coefficients between reduced hemoglobin and oxygenated hemoglobin as shown in Fig. 4. Thus, mother-derived nucleated red blood cells and fetus-derived nucleated red blood cells are sorted out using this difference. During measurement of an absorbance, a light wavelength region of 400 to 650 nm is applied. The absorbance is measured using at least one monochromatic light beam having a wavelength selected from this light wavelength region.

In addition, it is possible to reliably sort nucleated red blood cells by measuring the absorbance of each wavelength using a plurality of monochromatic light beams of different wavelengths during the measurement of an absorbance, and detecting the proportion of the measurement values of each of the wavelengths. It is preferable to perform sorting in accordance with the proportion of the absorbance of each wavelength preferably using an aspect in which monochromatic light having a wavelength selected from a light wavelength region of 400 to 500 nm is used, and more preferably using monochromatic light beam having a wavelength selected from a light wavelength region exceeding 450 nm and less than 480 nm and monochromatic light beam having a wavelength selected from a light wavelength region exceeding 550 nm and less than 575 nm. In addition, it is preferable to perform sorting in accordance with the proportion of the absorbance of each wavelength using monochromatic light beam having a wavelength selected from a light wavelength region exceeding 550 nm and less than 575 nm and monochromatic light beam having a wavelength selected from a light wavelength region exceeding 575 nm and less than 585 nm. It is possible to exclude absorbance errors between individual cells which are caused by the thickness of a cell, the area of a cell, or the like, by deriving the proportions of the absorbance which have been measured using two or more different types of monochromatic light. In these light wavelength regions, the difference in absorption coefficients is significant between oxygenated hemoglobin ($HbO_2$) and reduced hemoglobin (Hb) as shown in Fig. 4. Therefore, it is possible to reliably sort out mother-derived nucleated red blood cells and fetus-derived nucleated red blood cells using these light wavelength regions.

In addition, it is preferable to measure light wavelength regions in which sorting is performed in accordance with optical characteristics at wavelengths of each of the light wavelength regions by interposing a wavelength at which the magnitude of an absorption coefficient of reduced hemoglobin and the magnitude of absorption coefficient of oxygenated hemoglobin in the absorption coefficients in the graph shown in Fig. 4 are reversed. That is, the absorbance is measured at a wavelength of a first light wavelength region in which absorption coefficients of fetus-derived nucleated red blood cells (in which the proportion of oxygenated hemoglobin is high) are higher than those of mother-derived nucleated red blood cells (in which the proportion of oxygenated hemoglobin is low) and at a wavelength of a second light wavelength region in which absorption coefficients of mother-derived nucleated red blood cells are higher than those of fetus-derived nucleated red blood cells. It is possible to increase the difference between the proportion of fetus-derived nucleated red blood cells and the proportion of mother-derived nucleated red blood cells after obtaining the ratio of the absorbance measured at a wavelength of the first light wavelength region and the absorbance measured at a wavelength of the second light wavelength region for comparison, and therefore, it is possible to reliably sort out fetus-derived nucleated red blood cells.

Examples of the first light wavelength region include a light wavelength region exceeding 400 nm and less than 500 nm, exceeding 525 nm and less than 550 nm, and exceeding 575 nm and less than 585 nm. In addition, examples of the second light wavelength region include a light wavelength region exceeding 550 nm and less than 575 nm.

In addition, it is possible to perform sorting after measuring absorbances at a plurality of wavelengths in each of the light wavelength regions and using an average value of these absorbances. It is possible to reduce an influence of noise included in the measurement values of the absorbances, by performing the sorting in accordance with the average value of the absorbances.

«First Embodiment»

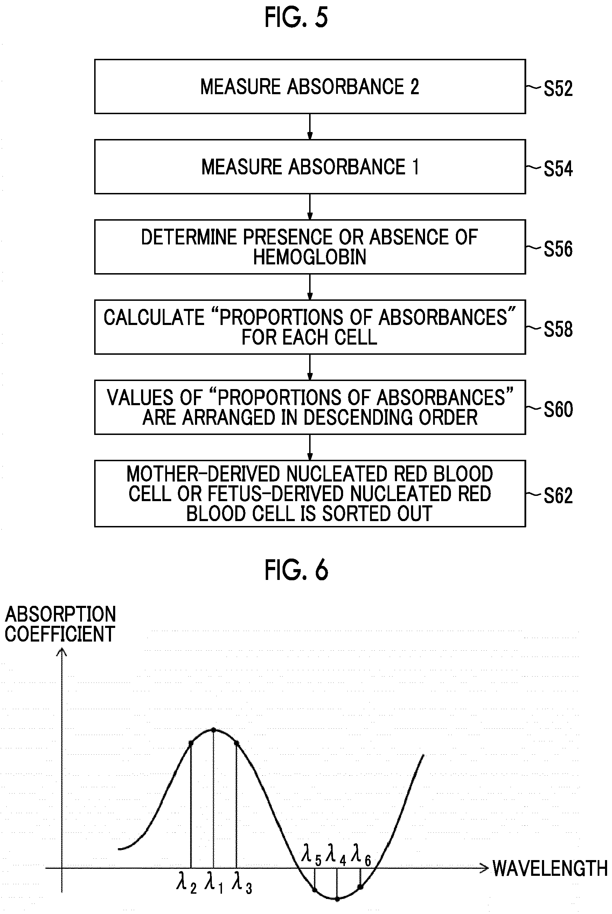

Fig. 5 is a flow chart showing a procedure of a first embodiment of sorting performed in accordance with optical characteristics. Fig. 6 is an explanatory view of a method for selecting a wavelength during measurement of an absorbance.

First, a plurality of nucleated red blood cells which become candidates of fetus-derived nucleated red blood cells are sorted out in accordance with the shape of a cell. Two types of wavelengths at which the absorbance is measured are selected in order to perform sorting on the plurality of nucleated red blood cells which become candidates of fetus-derived nucleated red blood cells which have been sorted out, in accordance with optical characteristics. For example, a wavelength $\lambda_1$ and a wavelength $\lambda_4$ in Fig. 6 are selected. An absorbance 2 is measured at one side which is a wavelength $\lambda_4$ (Step S52). Similarly, an absorbance 1 is measured at the other side which is a wavelength $\lambda_1$ (Step S54). As described above, after selecting two types of wavelengths $\lambda_1$ and $\lambda_4$ at which absorbances are to be measured, absorbances at wavelengths $\lambda_2$ and $\lambda_3$ which have been deviated from the wavelength $\lambda_1$ and at wavelengths $\lambda_5$ and $\lambda_6$ which have been deviated from the wavelength $\lambda_4$ are also respectively measured. It is possible to set an average value of the absorbances, which has been obtained by measuring the absorbances at wavelengths $\lambda_1$, $\lambda_2$, and $\lambda_3$, as the absorbance 1 and it is possible to set an average of the absorbances, which has been obtained by measuring the absorbances at wavelengths $\lambda_4$, $\lambda_5$, and $\lambda_6$, as the absorbance 2. In this manner, it is possible to reduce an influence of noise included in the measurement values of the absorbances by setting the average values of the absorbances measured at a plurality of wavelengths, as the absorbance 1 and the absorbance 2.

Next, discrimination of whether or not cells actually have hemoglobin is performed using the absorbances 1 and 2 (Step S56). The presence or absence of hemoglobin is performed through comparison with an absorbance with respect to a known wavelength of hemoglobin. If there is hemoglobin, it is determined that cells are nucleated red blood cells. Subsequently, proportions of absorbances (absorbance 1 / absorbance 2) for each nucleated red blood cell which becomes a candidate of a fetus-derived nucleated red blood cell are calculated and obtained (Step S58 in Fig. 5) and the values of the proportions of absorbances are sequentially arranged in a descending order (Step S60 in Fig. 5). Finally, the nucleated red blood cells which become candidates of fetus-derived nucleated red blood cells are sorted into mother-derived nucleated red blood cells and fetus-derived nucleated red blood cells by comparing the proportions of the measured absorbances of the nucleated red blood cells which become candidates of fetus-derived nucleated red blood cells, with a theoretical values of proportions which have been obtained from the measured wavelengths and the absorbances of oxygenated hemoglobin and reduced hemoglobin, based on the relationship between oxygenated hemoglobin and reduced hemoglobin at respectively selected wavelengths (Step S62 in Fig. 5).

<<Second Embodiment>>

As a method for sorting out fetus-derived nucleated red blood cells and mother-derived nucleated red blood cells through spectral characteristics, nucleated red blood cells to be sorted out are sorted into fetus-derived nucleated red blood cells or mother-derived nucleated red blood cells through comparison with reference red blood cells after setting red blood cells which have no nucleus existing around the nucleated red blood cells to be sorted out, as the reference red blood cells. It is possible to more reliably sort out fetus-derived nucleated red blood cells from the nucleated red blood cells to be sorted out through comparison between a proportion of an absorbance of a reference red blood cell and a proportion of an absorbance of a nucleated red blood cell to be sorted out, after obtaining a proportion of an absorbance of the reference red blood cell which has been measured using monochromatic light at the same wavelength as that of the nucleated red blood cell to be sorted out.

<Method for Selecting Reference Red Blood Cell>

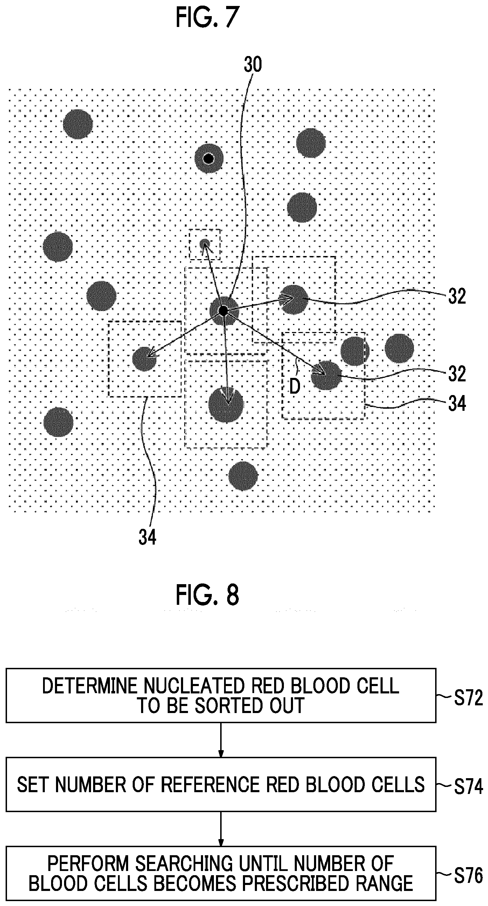

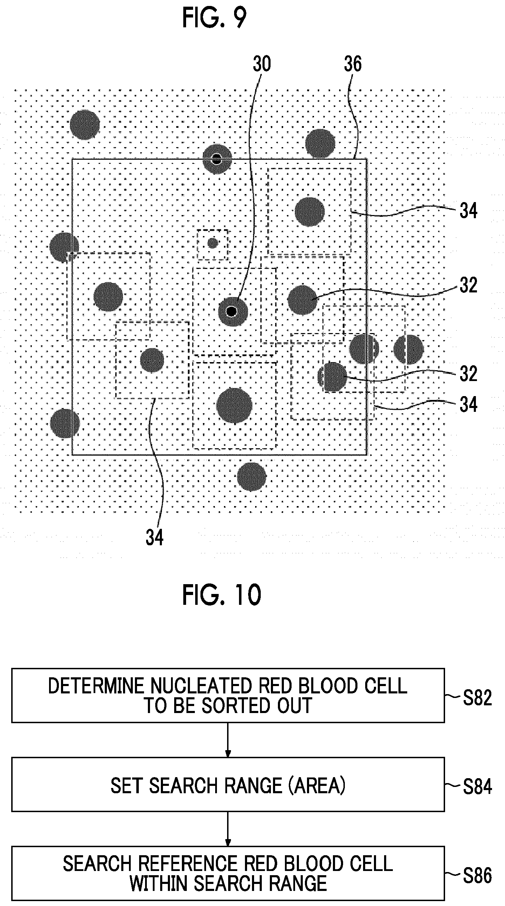

Selection of reference red blood cells can be performed through the following method. Figs. 7 to 10 are views illustrating a method for selecting reference red blood cells. Fig. 7 is a view illustrating a method for setting a search range based on the number of reference red blood cells. Fig. 8 is a flow chart showing a procedure of setting the search range based on the number of reference red blood cells. In addition, Fig. 9 is a view illustrating a method for setting a search range based on the area of a nucleated red blood cell which has been paid attention. Fig. 10 is a flow chart showing a procedure of setting the search range based on the area of a nucleated red blood cell which has been paid attention.

{Method for Setting Search Range Based on Number of Reference Red Blood Cells}

Figs. 7 and 8 are views illustrating the method for setting a search range based on the number of reference red blood cells. First, a nucleated red blood cell candidate 30 to be sorted out is determined (Step S72 in Fig. 8). The nucleated red blood cell candidate 30 to be sorted out can be determined in accordance with the shape as previously described. Next, the number of reference blood cells to be searched is set (Step S74 in Fig. 8). A case where the number of reference red blood cells to be searched is set to 5 is illustrated in Fig. 8. Next, reference red blood cells 32 are selected until the number of reference blood cells to be searched becomes a set number around the nucleated red blood cell candidate 30 to be sorted out (Step S76 in Fig. 8). The reference red blood cells 32 are selected in an ascending order of a distance D from the center of the nucleated red blood cell candidate 30 to be sorted out to the center of a reference red blood cell 32.

The center of the nucleated red blood cell candidate 30 can be set as the center of a circular shape in a case where the planar shape of the nucleated red blood cell candidate 30 is approximated to the circular shape. Similarly, the center of the reference red blood cell 32 can be set as the center of a circular shape in a case where the planar shape of the reference red blood cell 32 is approximated to the circular shape. The same principle also applies to the following description. In addition, reference red blood cells may be selected in an ascending order of the distance from the center of gravity of the nucleated red blood cell candidate 30 to the center of gravity of the reference red blood cell 32.

In Fig. 7, it has been described that the number of reference red blood cells selected is 5. However, the number of reference red blood cells selected is preferably 2 to 20. A standard which is different from the nucleated red blood cell candidate to be sorted out can be determined without variation by setting the number of reference red blood cells selected to be within the above-described range and obtaining and averaging optical information of the plurality of reference red blood cells. In addition, the number of reference red blood cells selected is more preferably set to be 3 to 10. A large number of reference red blood cells are preferable since it is possible to average optical information as the number of reference red blood cells is larger. However, it takes time to measure the reference red blood cells if the number of reference red blood cells is large, and therefore, it is preferable that the number of reference red blood cells is within the above-described range.

{Method for Setting Search Range Based on Area of Nucleated Red Blood Cell to be Sorted out}

Figs. 9 and 10 are views illustrating a method for setting a search range based on the area of a nucleated red blood cell to be sorted. Even in the method, a nucleated red blood cell candidate 30 to be sorted out is first determined similarly to the method for setting a search range based on the number of reference red blood cells (Step S82 in Fig. 10). Next, a search range 36 of reference red blood cells 32 is set (Step S84 in Fig. 10). In Fig. 9, the search range 36 is shown by a solid line. The search range 36 shown in Fig. 9 is set to 100 cell fractions (10 x 10 cell fractions) of the nucleated red blood cell candidate 30 to be sorted out.

It is preferable that the search range 36 is set to a square of which a side around the nucleated red blood cell candidate 30 to be sorted out is 10 cell fractions of the nucleated red blood cell candidate 30, or to a circular shape of which the diameter is 10 cell fractions. In addition, in a case where the search range 36 is set stepwise and there is no reference red blood cell in the search range 36, it is preferable to widen the search range 36. For example, in a case where the search range 36 is set to a square of which a side is 10 cell fractions of the nucleated red blood cell candidate 30, or to a circular shape of which the diameter is 10 cell fractions, if there is no reference red blood cell 32, it is preferable to determine reference red blood cells after further widening the search range up to a square of which a side is 20 cell fractions or a circular shape of which the diameter is 20 cell fractions.

Next, red blood cells having no nucleus within the search range 36 are searched as the reference red blood cells 32 (Step S86 in Fig. 10). The standard to be selected as the reference red blood cell 32 is determined based on whether or not the center or the center of gravity of the reference red blood cell 32 is within the search range 36.

The number of reference red blood cells 32 selected and the area of the search range 36 are not particularly limited, and can be arbitrarily set.

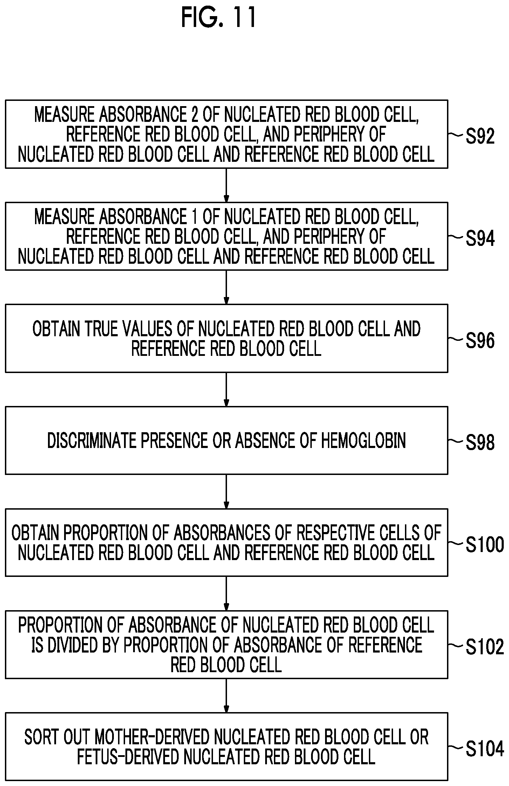

Fig. 11 is a flow chart showing a procedure of sorting out a nucleated red blood cell to be sorted out after selecting reference red blood cells 32.

After selecting the reference red blood cells 32, absorbances 2 of (1) a nucleated red blood cell candidate 30 to be sorted out, (2) reference red blood cells 32, and (3) a periphery 34 (shown by a broken line in Figs. 7 and 9) of the nucleated red blood cell candidate 30 to be sorted out and the reference red blood cells 32 are measured at selected wavelengths (Step S92 in Fig. 11). The periphery 34 of the nucleated red blood cell candidate 30 to be sorted out and the reference red blood cells 32 refers to a range of 9 cell fractions (3 x 3 cell fractions) of target red blood cells in Figs. 7 and 9. Similarly, absorbances 1 of (1) a nucleated red blood cell candidate 30 to be sorted out, (2) reference red blood cells 32, and (3) a periphery 34 of the nucleated red blood cell candidate 30 to be sorted out and the reference red blood cells 32 are measured at wavelengths different from those selected in Step S92 in Fig. 11 (Step S94 in Fig. 11).

After measuring the absorbances 1 and 2, (4) true values of the absorbances 1 and 2 of the nucleated red blood cell candidate 30 are obtained by subtracting an absorbance of the periphery 34 of the nucleated red blood cell candidate 30 from the absorbance of the nucleated red blood cell candidate 30. In addition, (5) true values of the absorbances 1 and 2 of the reference red blood cells 32 are obtained by subtracting an absorbance of a periphery 34 of each red blood cell of the reference red blood cells 32 from the absorbances of the reference red blood cells 32 (Step S96 in Fig. 11).

Discrimination of whether the nucleated red blood cell candidate 30 actually has hemoglobin is performed using absorbances 1 and 2 of the nucleated red blood cell candidate 30 (Step S98 in Fig. 11). The presence or absence of hemoglobin is discriminated through a known absorbance with respect to a wavelength of hemoglobin. With the presence of hemoglobin, it is determined that the nucleated red blood cell candidate 30 is a nucleated red blood cell.

Next, the proportion of the absorbance of a nucleated red blood cell and the proportion of the absorbance of a reference red blood cell are obtained (Step S100). The proportion of the absorbance of a nucleated red blood cell is obtained using "true value of absorbance 1 / true value of absorbance 2". In addition, the proportion of the absorbance of a reference red blood cell can be obtained using "average value of true value of absorbance 1 / average value of true value of absorbance 2" after obtaining an average value of true values of absorbances 1 of a plurality of reference red blood cells and an average value of true values of absorbances 2 of the plurality of reference red blood cells.

Finally, "proportion of absorbance of nucleated red blood cell / proportion of absorbance of reference red blood cell" is obtained (Step S102). Since the reference red blood cells are mother-derived nucleated red blood cells, there is a high possibility that the nucleated red blood cell may be a mother-derived nucleated red blood cell as the difference between the value of "proportion of absorbance of nucleated red blood cell / proportion of absorbance of reference red blood cell" and "1" is small and there is a high possibility that the nucleated red blood cell may be a fetus-derived nucleated red blood cell as the difference between the value thereof and "1" is large, and the nucleated red blood cell can be sorted into a fetus-derived nucleated red blood cell (Step S104). In this manner, it is possible to sort out a fetus-derived nucleated red blood cell by obtaining the "proportion of absorbance of nucleated red blood cell / proportion of absorbance of reference red blood cell" and prioritizing the possibility that a nucleated red blood cell in which the difference between the value and "1" is greatest is a fetus-derived nucleated red blood cell.

As the method for selecting reference red blood cells, the method for selecting reference red blood cells after setting a search range based on the number of reference red blood cells as shown in Figs. 7 and 8 and the method for selecting reference red blood cells after setting a search range based on the area of a nucleated red blood cell to be sorted out as shown in Figs. 9 and 10 have been exemplified. However, the method for selecting reference red blood cells is not limited thereto, and it is also possible to perform the sorting through a method in which these methods are combined. For example, in a case where the number of red blood cells included in a search range which has been determined using the area of a nucleated red blood cell to be sorted out exceeds the number of nucleated red blood cells which has been previously set, it is possible to measure all red blood cells or to measure red blood cells after selecting the number of red blood cells which has been previously set. As the selection method, a reference red blood cell of which the distance between the center of a nucleated red blood cell to be sorted out and the center of the reference red blood cell is short or a reference red blood cell of which the distance between the center of gravity of a nucleated red blood cell to be sorted out and the center of gravity of the reference red blood cell is short is sequentially selected as described above. In addition, in a case where the number of reference red blood cells within the search range is smaller than the number of reference red blood cells which has been previously set, the search range is enlarged at a point in time at which the small number of reference red blood cells is determined, and the reference red blood cells included in the increased area due to the enlargement of the search range are detected. It is preferable to repeat this operation until a sufficient number of red blood cells which has been previously determined is obtained.

In addition, in a case where sorting is performed through comparison with reference red blood cells, the method is not limited to a method for performing sorting using the proportion of absorbances measured at a plurality of wavelengths, and it is also possible to perform discrimination by comparing reference red blood cells with nucleated red blood cells to be sorted out after measuring an absorbance at a wavelength. Since a reference red blood cell is a mother-derived red blood cell, it is possible to discriminate that a nucleated red blood cell having a value of an absorbance close to that of a reference red blood cell is derived from a mother and a nucleated red blood cell having a value of an absorbance far from that of a reference red blood cell is derived from a fetus.

It is possible to sort nucleated red blood cells existing in maternal blood into fetus-derived nucleated red blood cells and mother-derived nucleated red blood cells through the sorting step using the above-described method.

<Amplification Step (Step S18)>

The amplification step is a step of amplifying nucleic acids contained in chromosomes of at least fetus-derived nucleated red blood cells which have been sorted out through the sorting step.

The fetus-derived nucleated red blood cells which have been sorted out through the sorting step are separated from a specimen using a micromanipulator. DNA is extracted from the cells which have been obtained by being separated from the specimen, and whole genome amplification is performed. The whole genome amplification can be performed using a commercially available kit.

Genome DNA obtained through elution from the obtained cells through cytolysis using a surfactant and a protein decomposition step using protease K or the like which are general methods is used for the whole genome amplification used in the present embodiment.

It is possible to use a reagent Single cell WGA kit (New England Biolabs) based on a polymerase chain reaction (PCR), a GenomePlex Single Cell Whole Genome Amplification kit (Sigma-Aldrich Co. LLC.), and Multiple Annealing and Looping-Based Amplification Cycles (MALBAC method) (published in WO2012/166425A2 as a whole genome amplification reagent. In addition, it is possible to similarly use a reagent GenomiPhi (GE Healthcare) and REPLI-g (QIAGEN) based on a chain-substituting DNA synthesis reaction. In the present embodiment, it is preferable to use a Single cell WGA kit (New England Biolabs).

It is preferable to check whether or not amplification is performed on an amplified product of DNA which has been obtained through whole genome amplification, through agarose gel cataphoresis. Furthermore, it is preferable to purify the whole genome amplification product using QIAquick PCR Purification Kit (QIAGEN).

In addition, it is preferable to measure the concentration of the amplified product of DNA which has been obtained through whole genome amplification using NANODROP (Thermo Fisher Scientific Inc.) and BIOANALYZER (Agilent Technologies).

In the amplification step, DNA which is a nucleic acid existing in a chromosome of at least a fetus-derived nucleated red blood cell is amplified. The number of fetus-derived nucleated red blood cells which are to be subjected to the amplification step may be at least one, and it is preferable to amplify nucleic acids which have been obtained from a plurality of fetus-derived nucleated red blood cells. Furthermore, selection of a chromosome of a mother-derived nucleated red blood cell, in which there is no numerical abnormality, as a standard for comparing the amounts of amplified products with each other is also a preferred aspect for determining a numerical abnormality of a chromosome of a fetus in a determination step below. In a case where the mother-derived nucleated red blood cell is compared with the standard, amplification of a nucleic acid of a chromosome of a mother-derived nucleated red blood cell which has been sorted out through the sorting step is also a preferred aspect.

<Definition Step (Step S20)>

The definition step is a step of defining the amount of an amplified product of at least a fetus-derived nucleated red blood cell which has been amplified through the amplification step.

(Determination of Number of Nucleic Acid Fragment)

The amount of an amplified product of DNA, which is identified as a cell of fetus-derived nucleated red blood cell, amplified through a polymerase chain reaction, and has a sequence of a region of 100 to 150 base pair (bp) which has been previously determined, with respect to a chromosome for which a numerical abnormality is to be inspected, is obtained using a sequencer. In the present invention, a chromosome of a fetus-derived nucleated red blood cell for which a numerical abnormality is to be inspected is preferably selected from the group consisting of chromosome 13, chromosome 18, chromosome 21, and x-chromosome.

<Determination Step (Step S22)>

The determination step is a step of determining the presence or absence of a numerical abnormality of a fetus-derived chromosome by comparing the amounts of amplified products of DNAs, which have been defined through the definition step, with each other.

A chromosome other than a chromosome for which a numerical abnormality is to be inspected is selected as a standard (or a reference) for determining the presence or absence of a numerical abnormality of a fetus-derived chromosome, and the amplification amount of an amplified product of DNA having a sequence of a region of 100 to 150 bp which has been previously determined is obtained using a sequencer. As a chromosome (reference chromosome) which becomes a standard, an aspect, in which at least one chromosome other than a chromosome for which a numerical abnormality is to be inspected is selected from chromosomes of fetus-derived nucleated red blood cells, or an aspect, in which a chromosome existing in a cell which is identified as a mother-derived nucleated red blood cell is selected, is selected. In the present embodiment, it is preferable to select a chromosome existing in a cell which is identified as a mother-derived nucleated red blood cell.

Next, whether or not there is a numerical abnormality in a chromosome of a fetus is determined from the ratio of the amount of an amplified product of DNA of a target chromosome of an inspection of a numerical abnormality to the amount of an amplified product of DNA of a reference chromosome. In a case where a fetus is in a normal condition, it is expected that the ratio of the amount of an amplified product of DNA of a fetus-derived chromosome for which a numerical abnormality is to be inspected to the amount of an amplified product of DNA of a reference chromosome may be approximately 1:1. In a case of trisomy which is a numerical abnormality in which there are three chromosomes, it is expected that the ratio may be 1.0:1.5 (or 2:3).

In addition, before the determination step, a distribution of results in which the ratios of the amounts of amplified products of DNAs of fetus-derived chromosomes to the amounts of amplified products of DNAs of mother-derived chromosomes, which have been collected from a plurality of pregnant mothers, in a case where each mother is pregnant with a normal fetus are obtained in plural times, and a distribution of results in which the ratios of the amounts of amplified products of fetus-derived DNAs to the amounts of amplified products of mother-derived DNAs of each mother who pregnant with a fetus with trisomy are obtained in plural times are previously obtained, and a cutoff value is set in a region where these two distributions do not overlap. Then, it is possible to determine whether or not there is a numerical abnormality by comparing the ratios of the amounts of the amplified products of DNAs with this cutoff value. In this case, it is possible to interpret the inspection results such that a fetus is normal if the ratio of the amount of an amplified product of DNA is less than or equal to the cutoff value, and there is a numerical abnormality which is trisomy if the ratio thereof is greater than or equal to the cutoff value.

[Examples]

(Example 1)

Hereinafter, the present invention will be described in more detail using examples exemplified below. However, the present invention is not limited to these examples.

(Acquisition Step [Peripheral Blood Sample Acquisition Step])

After adding 10.5 mg of sodium salts of ethylenediaminetetraacetic acid (EDTA) to a 7 mL blood collecting tube as an anticoagulant, 7 mL of peripheral blood was obtained in the blood collecting tube as volunteer blood after obtaining informed consent from a volunteer of a mother. Thereafter, blood was diluted using a physiological salt solution.

(Concentration Step [Concentration Step of Nucleated Red Blood Cell])

A liquid with a density of 1.070 g/mL and a liquid with a density of 1.095 g/mL were prepared using a Percoll liquid (registered trademark), and 2 ml of a liquid with a density of 1.095 g/mL was added to a bottom portion of a centrifuge tube. Continuously, 2 mL of a liquid with a density of 1.070 g/mL was made to slowly overlap the top of the liquid with a density of 1.095 g/mL so as not to disturb an interface. Thereafter, 11 mL of diluent of blood which had been collected in the above was slowly added to the top of the medium with a density of 1.070 g/mL in the centrifuge tube. Then, centrifugation was performed for 20 minutes at 2000 revolution per minute (rpm). Thereafter, the centrifuge tube was taken out and fractions which had been deposited between the solution with a density of 1.070 g/mL and the solution with a density of 1.090 g/mL were collected using a pipette. A first glass substrate was held by one hand and a drop of the fractions of blood which had been collected in this manner was placed at one end of the first glass substrate. Another glass substrate (second glass substrate) was held by the other hand and was brought into contact with the first glass substrate at 30 degrees. If the lower surface of the second glass substrate which is brought into contact with the fractions of blood, the fractions of blood spread into the space surrounded by the two sheets of glass due to a capillary phenomenon. Next, the first glass substrate was coated with the fractions of blood and the second glass substrate was made to be slid in a direction of a region opposite to the region of the first glass substrate, on which blood was placed, while maintaining the angle. Thereafter, the first glass substrate was sufficiently dried through air blowing for one hour. This first glass substrate was immersed in a MAY-Grunwald dyeing liquid for three minutes and was washed by being immersed in a phosphoric acid buffer solution. Thereafter, the first glass substrate was immersed in a GIEMSA dyeing liquid (which was diluted with a phosphoric acid buffer solution to make a concentration of 3%) for 10 minutes. Thereafter, the first glass substrate was dried after being washed with pure water. A plurality of glass substrates dyed in this manner were produced.

(Sorting Step Based on Shape of Cell)

In order to sort out a nucleated red blood cell which becomes a candidate of a fetus-derived nucleated red blood cell, from blood after being subjected to concentration with which the glass substrate was coated, an electric two-dimensional stage (hereinafter, denoted as an XY stage), a measurement system of an optical microscope including an objective lens and a CCD camera, a control unit including an XY stage control unit and a Z-direction control unit of controlling a vertical direction (hereinafter, denoted as a Z-direction) movement mechanism, and a control unit portion including an image input unit, an image processing unit, and an XY position recording unit. Blood cells which had been prepared as described above and with which the glass substrate was coated were placed on the XY stage and scanning was performed by being focused on the glass substrate, an image which had been obtained using an optical microscope was taken, and nucleated red blood cells which were target cells were searched through image analysis.

In the image analysis, the positions of cells in an X-direction and a Y-direction of the detected cells were recorded in a case where cells which satisfied the conditions of the following Formulas (1) and (2) were detected, a coordinate system consisting of two axes orthogonal to an image to be analyzed was set, and one axis of the two axes was set to an X-axis and the other of the two axes was set to a Y axis.

$$0.25 < (N / C) < 1.0 \qquad (1)$$

$$0.65 < (N / (L \times L)) < 0.785 \qquad (2)$$

Here, C is defined as the area (the area of a region indicating cytoplasm in an image used for analysis) of cytoplasm of a cell for which image analysis is performed, N is defined as the area (the area of a region indicating a nucleus of a cell in an analysis image) of a nucleus of a cell for which image analysis is performed, L is defined as the major axis or the diameter (the major axis of an elliptical shape in a case where a region indicating a nucleus of a cell in an analysis image is approximated to the elliptical shape, or the diameter of a circular shape in a case where a region indicating a nucleus in a cell in an analysis image is approximated to the circular shape) of a nucleus of a cell for which image analysis is performed, that is, the major axis of an elliptical shape or the diameter of a circular shape circumscribing a cell nucleus which has a complicated shape. A nucleated red blood cell which became a candidate of a fetus-derived nucleated red blood cell existing on a slide glass substrate satisfying Formulas (1) and (2) was selected and was set as a candidate of a fetus-derived nucleated red blood cell of the next step.

(Step of Sorting Nucleated Red Blood Cells Based on Optical Characteristics)

Analysis of optical characteristics was performed on ten nucleated red blood cells which became candidates of fetus-derived nucleated red blood cells and had been selected according to information of the shape using a microspectroscopic device. Ten nucleated red blood cells which became candidates of fetus-derived nucleated red blood cells on a slide glass substrate were specified, an absorbance 1 of monochromatic light having a wavelength of 460 nm and an absorbance 2 of $\mu$L having a wavelength of 560 nm were measured regarding one nucleated red blood cell out of the ten nucleated red blood cells to calculate the proportion of absorbances (absorbance 1 / absorbance 2). Next, five red blood cells having no nucleus were selected as reference red blood cells, which were at a position in the vicinity of the nucleated red blood cells, in an ascending order of the distance from the nucleated red blood cells, the proportion of absorbances (absorbance 1 / absorbance 2) of each reference red blood cell was calculated in the same manner, and an average value was calculated.

Calculation was similarly performed on remaining nine cells of the nucleated red blood cells which became candidates of fetus-derived nucleated red blood cells on the glass substrate through the same method. The (average value of proportions of absorbances of nucleated red blood cells / proportions of absorbances of reference red blood cells) was obtained from this calculation result, a cell in which the difference between this average value and "1" was greatest was regarded as a fetus-derived nucleated red blood cell and was set to a cell A, and a cell in which the difference between this average value and "1" was smallest was regarded as a mother-derived nucleated red blood cell and was set to a cell B.

(Cell Isolation Step)

The cells A and B which had been determined in the above-described step were collected using a micromanipulator.

(Amplification Step [DNA Amplification Step])

Whole genome amplification was performed using Single Cell WGA kit manufactured by New England Biolabs using the cell A of the nucleated red blood cell which was identified as being derived from a fetus and the cell B of the nucleated red blood cell which was identified as being derived from a mother to amplify a small amount of DNA in the cells into about a million times in accordance with description of a manual.

(Definition Step [Step of Defining Whether Cell Is Derived from Mother or Fetus])

It was possible to confirm that SNP of the cell A is different from that of the cell B through comparison of single nucleotide polymorphisms (SNP) (SNP ID: rs3751355, rs1799955, rs2297555, and rs9571740) of a C1QTNF9B gene region, a PCDH9 gene region, a BRCA2 gene region, and a MTRF1 gene region of chromosome 13 with each other using a genome analyzer MISEQ manufactured by Illumina, Inc. after equally dividing the whole genome amplification product which had been amplified from each cell and using one of the divided whole genome amplification products. A cell C which was expected to be a white blood cell was separately collected using a micromanipulator and SNP of the cell C was checked similarly to those of the cell A and cell B. As a result, it was confirmed that SNP of the cell C was coincident with SNP of the cell B. From the above, it was confirmed that the cell A was a fetus-derived nucleated red blood cell and the cell B was a mother-derived nucleated red blood cell.

(Determination Step [Step of Determining Numerical Abnormality])

It was discriminated whether the fetus-derived nucleated red blood cell was trisomy 21 using one of the remaining amplified products of DNA which had been amplified from each of the cells A and B and equally divided. It was possible to detect trisomy 21 by comparing the number of sequence reads which had been calculated in an exon portion of chromosome 21 in the each of the cells A and B with the number of sequence reads of the exon portion which had been calculated using a genome analyzer HISEQ manufactured by Illumina, Inc. after concentrating only the exon portion of each cell in accordance with description of a manual using a SURESELECT TARGET ENRICH system (HUMAN ALL EXON V5 manufactured by Agilent Technologies) using the amplified products of DNA which have been obtained from each of the cells A and B.
The effect of the present invention was confirmed since it was found that it was possible to identify fetus-derived nucleated red blood cells from blood obtained from a pregnant mother and to analyze a numerical abnormality of a chromosome of a fetus.

(Example 2)

The effect of the present invention was confirmed in the same manner as in Example 1 except that a glass substrate was produced by performing through a method described below, instead of the (Concentration Step [Concentration Step of Nucleated Red Blood Cell]) in Example 1.

(Concentration Step [Concentration Step of Nucleated Red Blood Cell])

A liquid with a density of 1.095 was prepared using a Histopaque liquid (registered trademark), and 3 mL of a liquid with a density of 1.095 was added to a bottom portion of a centrifuge tube. Thereafter, 12 mL of diluent of blood which had been collected was slowly added to the top of the medium with a density of 1.095 in the centrifuge tube. Then, centrifugation was performed for 20 minutes at 2000 rpm. Thereafter, the centrifuge tube was taken out and fractions between the solution with a density of 1.095 and plasma were collected using a pipette. A first glass substrate was held by one hand and a drop of the fractions of blood which had been collected in this manner was placed at one end of the first glass substrate. Another glass substrate 2 (second glass substrate) was held by the other hand and one end of the second glass substrate was brought into contact with the first glass substrate at 30 degrees. If the lower surface of the second glass substrate which is brought into contact with the fractions of blood, the fractions of blood spread into the space surrounded by the two sheets of glass due to a capillary phenomenon. Next, the first glass substrate was coated with the fractions of blood and the second glass substrate was made to be slid in a direction of a region opposite to the region of the first glass substrate, on which blood was placed, while maintaining the angle. Thereafter, the first glass substrate was sufficiently dried through air blowing for one hour. This first glass substrate was immersed in a MAY-Grunwald dyeing liquid for three minutes and was washed by being immersed in a phosphoric acid buffer solution. Thereafter, the first glass substrate was immersed in a GIEMSA dyeing liquid (which was diluted with a phosphoric acid buffer solution to make a concentration of 3%) for 10 minutes. Thereafter, the first glass substrate was dried after being washed with pure water. A plurality of glass substrates dyed in this manner were produced.

(Example 3)

The effect of the present invention was confirmed by performing a test in the same manner as in Example 1 except that:

the presence or absence of a PCR amplified product of a Y chromosome was confirmed through a general agarose gel electrophoresis method by performing PCR using a Multiplex PCR Assay Kit manufactured by TAKARA BIO INC. using PCR primer which had been produced in a sex-determining factor (SRY) gene region of the Y chromosome, instead of the method for defining whether cells are derived from a mother or a fetus, which was performed in Example 1, using cells obtained from another mother than the mother in Example 1; and

it was confirmed that cells, which had been obtained from the other mother and identified as being derived from a fetus, were fetus-derived cells of a male infant in the sorting step of nucleated red blood cells of a fetus in Example 1.

Explanation of References

30: nucleated red blood cell candidate
32: reference red blood cell
34: periphery
36: search range

**Claims**

1. A method for inspecting a chromosome of a fetus, the method comprising:

   a collection step of obtaining maternal blood from a pregnant mother;
   a concentration step of concentrating nucleated red blood cells in the maternal blood;
   a sorting step of sorting the nucleated red blood cells in the maternal blood, in which the nucleated red blood cells are concentrated through the concentration step, into mother-derived nucleated red blood cells and fetus-derived nucleated red blood cells in accordance with shapes of nuclei and spectral characteristics at wavelengths included in a light wavelength region of 400 to 650 nm;
   an amplification step of amplifying a nucleic acid of a chromosome of at least the fetus-derived nucleated red blood cells;
   a definition step of defining an amount of the amplified product of at least the fetus-derived nucleated red blood cells which has been amplified through the amplification step; and
   a determination step of determining presence or absence of a numerical abnormality of the fetus-derived chromosome through comparison of the defined amount of the amplified product with an amount of an amplified product of a chromosome of a mother or an amount of an amplified product of a chromosome of a fetus which is known that there is no abnormality.

2. The method for inspecting a chromosome of a fetus according to claim 1,

   wherein the sorting step includes a first sorting step of sorting out a candidate of a nucleated red blood cell in accordance with at least the shapes of the nuclei, and a second sorting step of sorting out the candidate of the nucleated red blood cell which has been sorted out through the first sorting step, in accordance with the spectral characteristics.

3. The method for inspecting a chromosome of a fetus according to claim 2,

   wherein the first sorting step includes a step of selecting a candidate of a nucleated red blood cell satisfying Formulas 1 and 2, in a case where an area of cytoplasm of a cell which becomes the candidate of the nucleated red blood cell is set to C, an area of a nucleus of a cell which becomes the candidate of the nucleated red blood cell is set to N, and a length of a diameter of a circular shape circumscribing the nucleus of the cell which becomes the candidate of the nucleated red blood cell or a length of a major axis of an elliptical shape thereof is set to L.

$$0.25 < N / C < 1.0 \qquad \text{Formula 1}$$

$$0.65 < N / (L \times L) < 0.785 \qquad \text{Formula 2}$$

**4.** The method for inspecting a chromosome of a fetus according to claim 3,

wherein the first sorting step further includes two steps including a first step of extracting cells possibly having a nucleus, and a second step of sorting out the candidate of the nucleated red blood cell from the cells extracted in the first step using Formulas 1 and 2.

**5.** The method for inspecting a chromosome of a fetus according to any one of claims 1 to 4,

wherein the spectral characteristics include an extinction coefficient.

**6.** The method for inspecting a chromosome of a fetus according to claim 5,

wherein, in the sorting step, an absorbance of the nucleated red blood cell is measured at each of at least two or more wavelengths including a wavelength in a first light wavelength region, in which an absorbance of the fetus-derived nucleated red blood cell exceeds an absorbance of the mother-derived nucleated red blood cell, and a wavelength in a second light wavelength region in which the absorbance of the mother-derived nucleated red blood cell exceeds the absorbance of the fetus-derived nucleated red blood cell.

**7.** The method for inspecting a chromosome of a fetus according to claim 5 or 6,

wherein, in the sorting step, a possibility of being a fetus-derived nucleated red blood cell is prioritized based on difference in the absorbances of the mother-derived nucleated red blood cell and the fetus-derived nucleated red blood cell which have been measured at each of two or more wavelengths.

**8.** The method for inspecting a chromosome of a fetus according to claim 7,

wherein the first light wavelength region is a wavelength region exceeding 450 nm and less than 480 nm, and the second light wavelength region is a wavelength region exceeding 550 nm and less than 575 nm.

**9.** The method for inspecting a chromosome of a fetus according to any one of claims 1 to 8,

wherein the concentration step is performed through density gradient centrifugation.

**Amended claims under Art. 19.1 PCT**

**1.** (Amended) A method for inspecting a chromosome of a fetus, the method comprising:

a collection step of obtaining maternal blood from a pregnant mother;
a concentration step of concentrating nucleated red blood cells in the maternal blood;
a sorting step of sorting the nucleated red blood cells in the maternal blood, in which the nucleated red blood cells are concentrated through the concentration step, into mother-derived nucleated red blood cells and fetus-derived nucleated red blood cells in accordance with shapes of nuclei and spectral characteristics at wavelengths included in a light wavelength region of 400 to 650 nm;
an amplification step of amplifying a nucleic acid of a chromosome of at least the fetus-derived nucleated red blood cells;
a definition step of defining an amount of the amplified product of at least the fetus-derived nucleated red blood cells which has been amplified through the amplification step; and
a determination step of determining presence or absence of a numerical abnormality of the fetus-derived chromosome through comparison of the defined amount of the amplified product with an amount of an amplified product of a chromosome of a mother or an amount of an amplified product of a chromosome of a fetus which is known that there is no abnormality, wherein
the sorting step includes a first sorting step of sorting out a candidate of a nucleated red blood cell in accordance with at least the shapes of the nuclei, and a second sorting step of sorting out the candidate of the nucleated red blood cell which has been sorted out through the first sorting step, in accordance with the spectral characteristics, and

the first sorting step includes a step of selecting a candidate of a nucleated red blood cell satisfying Formulas 1 and 2, in a case where an area of cytoplasm of a cell which becomes the candidate of the nucleated red blood cell is set to C, an area of a nucleus of a cell which becomes the candidate of the nucleated red blood cell is set to N, and a length of a diameter of a circular shape circumscribing the nucleus of the cell which becomes the candidate of the nucleated red blood cell or a length of a major axis of an elliptical shape thereof is set to L.

$$0.25 < N / C < 1.0 \qquad \text{Formula 1}$$

$$0.65 < N / (L \times L) < 0.785 \qquad \text{Formula 2}$$

**2.** Cancelled)

**3.** Cancelled)

**4.** (Amended) The method for inspecting a chromosome of a fetus according to claim 1,

wherein the first sorting step further includes two steps including a first step of extracting cells which possibly has a nucleus, and a second step of sorting out the candidate of the nucleated red blood cell from the cells extracted in the first step using Formulas 1 and 2.

**5.** (Amended) The method for inspecting a chromosome of a fetus according to claim 1 or 4,

wherein the spectral characteristics include an absorption coefficient.

**6.** The method for inspecting a chromosome of a fetus according to claim 5,

wherein, in the sorting step, an absorbance of the nucleated red blood cell is measured at each of at least two or more wavelengths including a wavelength in a first light wavelength region, in which an absorbance of the fetus-derived nucleated red blood cell exceeds an absorbance of the mother-derived nucleated red blood cell, and a wavelength in a second light wavelength region in which the absorbance of the mother-derived nucleated red blood cell exceeds the absorbance of the fetus-derived nucleated red blood cell.

**7.** The method for inspecting a chromosome of a fetus according to claim 5 or 6,

wherein, in the sorting step, a possibility of being a fetus-derived nucleated red blood cell is prioritized based on difference in the absorbances of the mother-derived nucleated red blood cell and the fetus-derived nucleated red blood cell which have been measured at each of two or more wavelengths.

**8.** The method for inspecting a chromosome of a fetus according to claim 7,

wherein the first light wavelength region is a wavelength region exceeding 450 nm and less than 480 nm, and the second light wavelength region is a wavelength region exceeding 550 nm and less than 575 nm.

**9.** (Amended) The method for inspecting a chromosome of a fetus according to any one of claims 1, 4, 5, 6, 7 and 8,

wherein the concentration step is performed through density gradient centrifugation.

**Statement under Art. 19.1 PCT**

The contents of the claims 2 and 3 are incorporated into the claim 1.
The term of "light-absorption coefficient" is corrected to "absorption coefficient" in the claim 5.
The claims 4, 5 and 9 are changed so as not to depend from the claims 2 and 3 due to the cancellation of the claims 2 and 3.

The claims 6 to 8 are unchanged.

FIG. 1

```
┌─────────────────────────────┐
│      COLLECTION STEP        │─~S12
└─────────────────────────────┘
              │
              ▼
┌─────────────────────────────┐
│    CONCENTRATION STEP       │─~S14
└─────────────────────────────┘
              │
              ▼
┌─────────────────────────────┐
│       SORTING STEP          │─~S16
└─────────────────────────────┘
              │
              ▼
┌─────────────────────────────┐
│    AMPLIFICATION STEP       │─~S18
└─────────────────────────────┘
              │
              ▼
┌─────────────────────────────┐
│      DEFINITION STEP        │─~S20
└─────────────────────────────┘
              │
              ▼
┌─────────────────────────────┐
│    DETERMINATION STEP       │─~S22
└─────────────────────────────┘
```

# FIG. 2

S42

IS SUBJECT CELL?
(CLOSED BY LINE?)  →  No

↓ Yes

S44

IS THERE NUCLEUS?
(IS THERE DOT-SHAPED
SUBSTANCE?)  →  No

↓ Yes

S46

IS SIZE OF NUCLEUS OF
CELL WITHIN PRESCRIBED
RANGE?  →  No

↓ Yes

S48

IS SHAPE OF NUCLEUS
WITHIN PRESCRIBED
RANGE?  →  No

↓ Yes

SELECT

DO NOT SELECT

# FIG. 3A

# FIG. 3B

FIG. 4

## FIG. 5

| | |
|---|---|
| MEASURE ABSORBANCE 2 | ~S52 |

↓

| | |
|---|---|
| MEASURE ABSORBANCE 1 | ~S54 |

↓

| | |
|---|---|
| DETERMINE PRESENCE OR ABSENCE OF HEMOGLOBIN | ~S56 |

↓

| | |
|---|---|
| CALCULATE "PROPORTIONS OF ABSORBANCES" FOR EACH CELL | ~S58 |

↓

| | |
|---|---|
| VALUES OF "PROPORTIONS OF ABSORBANCES" ARE ARRANGED IN DESCENDING ORDER | ~S60 |

↓

| | |
|---|---|
| MOTHER-DERIVED NUCLEATED RED BLOOD CELL OR FETUS-DERIVED NUCLEATED RED BLOOD CELL IS SORTED OUT | ~S62 |

## FIG. 6

ABSORPTION COEFFICIENT

WAVELENGTH

$\lambda_2\ \lambda_1\ \lambda_3$ $\lambda_5 \lambda_4 \lambda_6$

## FIG. 7

## FIG. 8

```
┌─────────────────────────────────────────┐
│ DETERMINE NUCLEATED RED BLOOD CELL       │──S72
│         TO BE SORTED OUT                  │
└─────────────────────────────────────────┘
                    │
                    ▼
┌─────────────────────────────────────────┐
│ SET NUMBER OF REFERENCE RED BLOOD CELLS  │──S74
└─────────────────────────────────────────┘
                    │
                    ▼
┌─────────────────────────────────────────┐
│    PERFORM SEARCHING UNTIL NUMBER OF      │──S76
│ BLOOD CELLS BECOMES PRESCRIBED RANGE      │
└─────────────────────────────────────────┘
```

# FIG. 9

# FIG. 10

DETERMINE NUCLEATED RED BLOOD CELL TO BE SORTED OUT — S82

SET SEARCH RANGE (AREA) — S84

SEARCH REFERENCE RED BLOOD CELL WITHIN SEARCH RANGE — S86

# FIG. 11

MEASURE ABSORBANCE 2 OF NUCLEATED RED BLOOD CELL, REFERENCE RED BLOOD CELL, AND PERIPHERY OF NUCLEATED RED BLOOD CELL AND REFERENCE RED BLOOD CELL ~S92

↓

MEASURE ABSORBANCE 1 OF NUCLEATED RED BLOOD CELL, REFERENCE RED BLOOD CELL, AND PERIPHERY OF NUCLEATED RED BLOOD CELL AND REFERENCE RED BLOOD CELL ~S94

↓

OBTAIN TRUE VALUES OF NUCLEATED RED BLOOD CELL AND REFERENCE RED BLOOD CELL ~S96

↓

DISCRIMINATE PRESENCE OR ABSENCE OF HEMOGLOBIN ~S98

↓

OBTAIN PROPORTION OF ABSORBANCES OF RESPECTIVE CELLS OF NUCLEATED RED BLOOD CELL AND REFERENCE RED BLOOD CELL ~S100

↓

PROPORTION OF ABSORBANCE OF NUCLEATED RED BLOOD CELL IS DIVIDED BY PROPORTION OF ABSORBANCE OF REFERENCE RED BLOOD CELL ~S102

↓

SORT OUT MOTHER-DERIVED NUCLEATED RED BLOOD CELL OR FETUS-DERIVED NUCLEATED RED BLOOD CELL ~S104

## INTERNATIONAL SEARCH REPORT

| International application No. |
| --- |
| PCT/JP2015/067711 |

**A. CLASSIFICATION OF SUBJECT MATTER**
*C12Q1/68*(2006.01)i, *C12Q1/04*(2006.01)i, *G01N21/27*(2006.01)i, *G01N33/48*
(2006.01)i, *G01N33/49*(2006.01)i

According to International Patent Classification (IPC) or to both national classification and IPC

**B. FIELDS SEARCHED**

Minimum documentation searched (classification system followed by classification symbols)
C12Q1/68, C12Q1/04, G01N21/27, G01N33/48, G01N33/49

Documentation searched other than minimum documentation to the extent that such documents are included in the fields searched

| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/MEDLINE/EMBASE/BIOSIS(STN),
DWPI(Thomson Innovation)

**C. DOCUMENTS CONSIDERED TO BE RELEVANT**

| Category* | Citation of document, with indication, where appropriate, of the relevant passages | Relevant to claim No. |
| --- | --- | --- |
| X/A | WO 2013/075100 A1 (CELLSCAPE CORP.), 23 May 2013 (23.05.2013), & JP 2014-533509 A & US 2013/0130265 A1 & US 2013/0130929 A1 & US 2013/0130930 A1 & EP 2780465 A | 1,2,5-9/3,4 |
| A | Etsuko ICHIDA, "Auto-extraction of Nucleated Red Blood Cells from Massive Microscopy Images", IEICE Technical Report, 2008, vol.107, no.461, pages 291 to 296 | 3,4 |
| A | JP 2002-514304 A (APPLIED IMAGING CORP.), 14 May 2002 (14.05.2002), & US 6169816 B1 & WO 1998/052018 A1 & EP 981732 A | 3,4 |

| ☒ | Further documents are listed in the continuation of Box C. | ☐ | See patent family annex. |
| --- | --- | --- | --- |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 14 August 2015 (14.08.15) | 25 August 2015 (25.08.15) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

International application No.

PCT/JP2015/067711

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-254042 A  (Fuji Xerox Co., Ltd.), 27 December 2012 (27.12.2012), & US 2014/0092228 A1    & WO 2012/169088 A1 & EP 2719754 A1 | 3,4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010525832 A
- JP 2002514304 A
- JP 58115346 A
- JP S58115346 A
- JP 2014014485 A
- JP 2004531271 A
- WO 2012023298 A
- JP 2014001485 A
- WO 2012166425 A2

### Non-patent literature cited in the description

- **WAKAKO SUZUKI et al.** Enrichment and Automated Detection of NRBC from Maternal Blood. The Showa University Society, 2012, vol. 72, 471-478
- **D. CHA et al.** A simple and sensitive erythroblast scoring system to identify fetal cells in maternal blood. *Prenat Diagn,* 2003, vol. 23, 68-73